(19) Europäisches Patentamt
European Patent Office
Office européen des brevets

(11) EP 1 665 939 A1

(12) EUROPEAN PATENT APPLICATION
published in accordance with Art. 158(3) EPC

(43) Date of publication:
**07.06.2006 Bulletin 2006/23**

(21) Application number: **04773106.2**

(22) Date of filing: **15.09.2004**

(51) Int Cl.:
***A23L 1/221*** (1974.07) ***A23L 1/227*** (1974.07)
***A23J 3/04*** (1990.01) ***A23J 3/34*** (1990.01)
***A23L 1/313*** (1974.07)

(86) International application number:
**PCT/JP2004/013443**

(87) International publication number:
**WO 2005/027657 (31.03.2005 Gazette 2005/13)**

(84) Designated Contracting States:
**CH DE FR GB IT LI**

(30) Priority: **19.09.2003 JP 2003328710**

(71) Applicants:
- **Amino Japan Co., Ltd.
Kamimashiki-gun,
Kumamoto 861-2202 (JP)**
- **Tomoe Food Research Co., Ltd.
Kumamoto-shi,
Kumamoto 860-0004 (JP)**

(72) Inventors:
- **MAEDA, Masamichi,
Ind. Technology Ctr. of Nagasaki
Ohmura-shi,
Nagasaki 856-0026 (JP)**
- **NODA, Y.,
Fukuoka-Pref. Shoyu Jozo Kyodo Kumiai
Chikushino-shi,
Fukuoka 818-0014 (JP)**
- **MURATA, Hisao,
c/o Tomoe Food Research Co., Ltd.
Kumamoto-shi,
Kumamoto 860-0004 (JP)**

(74) Representative: **Samson & Partner
Widenmayerstraße 5
D-80538 München (DE)**

# (54) PROCESS FOR PRODUCING PROTEIN HYDROLYSATE AND PROTEIN HYDROLYSATE

(57) It is intended to provide a process for producing a highly safe seasoning by highly decomposing meat or a bone residue remaining after taking meat (in particular, chicken meat, chicken bone or culled chicken) with enzymes. It is also intended to utilize nitrogen at an elevated ratio compared with the existing low nitrogen utilization level. In decomposing protein in chicken meat, culled chicken or chicken bone, muscular protein alone is decomposed by using an autolyzing enzyme or an autolyzing enzyme with an enzymatic agent containing peptidase. In the case of using chicken meat as the raw material, it contains trace constituents of meat (taurin, anserine, carnosine, creatine, etc.) and the content of Hyp (oxyproline) is controlled to 0.5% by mass or less to the protein. In the case of using culled chicken or chicken bone as the raw material, it contains trace constituents of meat (taurin, anserine, carnosine, creatine, etc.) and the content of Hyp (oxyproline) is controlled to 1.0% by mass or less to the protein.

FIG. 4

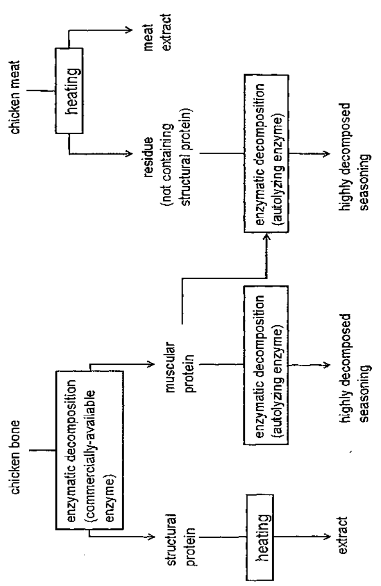


EP 1 665 939 A1

## Description

TECHNICAL FIELD

The present invention relates to a technique for producing a safe protein hydrolysate by hydrolyzing with enzymes protein of meat or a bone residue remaining after taking meat.

BACKGROUND ART

In agents used for seasoning food, there are chemical seasonings such as sodium glutamate or sodium inosinate and natural seasonings mainly produced from animals or plants. A large proportion of natural seasonings comprises extract seasonings and amino acid seasonings. Extract seasonings are produced from condensed extracts of stock farm products, vegetables, marine products, and the like. Amino acid seasonings are protein hydrolysates produced by decomposing protein into amino acids.

As processes for decomposing protein into amino acids, there are acid digestions using hydrochloric acid and enzymatic digestions. Amino acid seasonings produced by decomposing vegetable protein with hydrochloric acid are called HVP (Hydrolyzed Vegetable Protein) while those produced by decomposing animal protein with hydrochloric acid are called HAP (Hydrolyzed Animal Protein), both of which are widely used as food seasonings. The raw materials of HVP and HAP are vegetable protein of defatted soybeans and animal protein such as protein of fish or bone residues remaining after taking meat, respectively. HVP and HAP, which are produced by decomposition with hydrochloric acid, enable high decomposition at a low cost and have strong umami. However, reactions of hydrochloric acid with fat components can generate toxic chlorohydrin or mutagenic compounds and causes safety concerns.

On the other hand, enzymatic digestions, which use enzymes to decompose protein instead of hydrochloric acid, provide safe seasonings. However, without high decomposition (amination ratios: 55% or higher), appropriate seasonings are not available because of the bitter taste caused by generation of peptides. High decomposition with enzyme digestions entails technical difficulties and requires a large amount of enzyme, which often leads to poor cost performance. Here, an amination ratio is one of the indicators which show a decomposition rate of protein, and a higher amination ratio means that decomposition has proceeded. An amination ratio (%) is expressed in the equation: formole nitrogen (F-N) /total nitride (T-N) $\times$ 100.

A large number of other processes have been proposed to decompose protein using enzymes or rice malt whereas they are mostly for vegetable protein (mainly wheat protein). A typical technique for highly decomposing vegetable protein is a method of producing soy sauce, which has already been commercialized. As for animal protein, however, a high decomposition is deemed to be difficult. There are few seasonings produced by decomposing meat or bone with enzymes at present, and techniques for the methods have not yet been established.

For example, the patent document 1 describes a seasoning produced by dissolving and recovering muscular protein from bone or the like with enzymes, followed by mixing the resultant with rice malt. This technique is effective for elevating a nitrogen utilization ratio. However, during the decomposition and fermentation steps, a large amount of salt added to prevent putrefaction hinders enzymatic activity, which consequently extends the time for decomposition and fermentation. In addition, the amination ratio is only 33.0% which is problematically low.

Most of the extract seasonings are, in the case of muscular protein, extracts produced by thermal extraction with water (hydrothermal extraction) from meat. Hydrothermal extraction of meat leaves a large amount of residue, which is not utilized effectively. A process of decomposing the residue with rice malt is known as described in the patent document 2, for example. A process for decomposing the residue with enzymes has also been studied whereas any process for high decomposition with enzymes at a low cost has neither been established nor commercialized. Accordingly, instead of using meat which is expensive, bone remaining after taking meat is used in many cases, by adding water and heating the bone for extraction. For example, extract from chicken bone is called chicken extract, and extract from pork bone called pork extract, both of which are being widely used as broth.

Meat is roughly divided into muscular protein (the protein forming muscle fibers, such as actomyosin, miogen, and globulin) and structural protein (the protein forming connective tissues, such as collagen and elastin) (See Table 1: Complete protein content in meat, "Meat Chemistry", 1964, p.163). Muscular protein is partially soluble in water or salt solution and solidifies by heating. On the other hand, collagen, which is a main component of structural protein, is insoluble in water, but thermally decomposed by heating into gelatin and dissolves. The patent document 3 discloses a method of preparing a seasoning by decomposing gelatin with enzymes and separating it with a membrane.

[Table 1]

| Kind of Meat | Portion | Protein Content (% in total protein) | |
|---|---|---|---|
| | | Complete (muscular protein) | Incomplete (structural protein) |
| Beef | Back | 84 | 16 |
| | Breast | 72 | 28 |
| | Shank | 44 | 56 |
| Lamb | Upper loin | 80 | 20 |
| | Breast | 73 | 27 |
| | Shank | 50 | 50 |
| Pork | Back | 91 | 9 |
| | Ham | 88 | 12 |
| Chicken | Breast | 92 | 8 |

Patent document 1: Publication of Japanese Patent No. 2960864
Patent document 2: Publication of Japanese Patent No. 2631200
Patent document 3: Unexamined Japanese Patent Publication No. 5-84050
Non-patent document 1: Saburo Kanno, "Utilization of Bone as Food", Monthly Food Chemical, July, K. K. Shokuhin Kagaku Shinbunsha, July 1, 1991, p. 69
Non-patent document 2: J. O. Opiacha, M. G Mast and J. H. MacNeil, "A Research Note In-Vitro Protein Digestibility of Dehydrated Protein Extract from Poultry Bone Residue", Journal of Food Science, U.S.A., Institute of Food Technologists, 1991, Vol. 56, No. 6, p.p. 1751-1752

DISCLOSURE OF THE INVENTION

PROBLEMS TO BE SOLVED BY THE INVENTION

In hydrothermal extraction, trace constituents in muscular protein (amino acids, nucleic acids, etc.) are extracted to provide extract with rich flavor, whereas muscular protein itself solidified by heating is not utilized. On the contrary, collagen, which is structural protein, is gelatinized by heating and dissolves while gelatin itself has feeling of thickness but less tasty.

As described above, in hydrothermal extraction, muscular protein is not utilized, which holds nitrogen utilization ratios at a lower level. Furthermore, since extract from bone contains gelatin as its principal component, it only exhibits weak taste. In addition, it is not possible to highly decompose extract produced by hydrothermal extraction from chicken bone with commercially-available enzymes. As examples thereof, analytical values of the extracts (recovered proteins) from chicken meat and bone with heating are shown in Table 2 below.

[Table 2]

| | | | Chicken meat | Chicken bone |
|---|---|---|---|---|
| Raw material | Protein content | % | 21.3 | 15.3 |
| | Oxyproline (Hyp) % | | 0.162 | 0.63 |
| | Oxyproline (Hyp)/ Protein (C-P) | % | 0.76 | 4.1 |
| | Gelatin | % | 1.62 | 6.3 |
| | Gelatin/Protein | % | 7.2 | 43.8 |

Table continued

| Extraction method | | | 100°C 1 hr | 115°C 30 min | 100°C 2 hrs | 115°C 1 hr |
|---|---|---|---|---|---|---|
| Recovered protein | Recovery rate of protein (protein/ material protein) | % | 12.1 | 14.2 | 22.2 | 32.7 |
| | Oxyproline (Hyp) | % | 0.063 | 0.075 | 0.214 | 0.333 |
| | Gelatin | % | 0.63 | 0.73 | 2.14 | 3.33 |
| | Gelatin/Protein | % | 24.3 | 26.6 | 62.9 | 66.6 |

where

$$\text{protein (C-P (Crude-Protein))} = \text{total nitrogen (T-N)} \times 6.25$$

$$\text{gelatin} = \text{oxyproline (Hyp)} \times 10 \text{ (provisional)}$$

Oxyproline (hydroxyproline: Hyp) is an amino acid specifically contained in collagen. The amount of collagen or gelatin can be determined by measuring oxyproline.

An object of the present invention is to obtain a highly safe hydrolysate of protein by highly decomposing with enzymes, at an amination ratio of 55% or more, protein of meat or a bone residue remaining after taking meat, as well as to utilize nitrogen at an elevated ratio compared with the existing low nitrogen utilization level.

MEANS TO SOLVE THE PROBLEMS

In order to solve the above-described problems, the inventors have made a variety of researches and reached the following findings to accomplish the present invention.

1. Gelatin components which are structural protein of meat are hardly decomposed with enzymes while muscular protein alone (protein soluble in water or salt water) separated from structural protein is decomposed more easily.
2. Meat or bone contains autolyzing enzymes and various kinds of peptidase as well as proteinase, which enables high-level decomposition only with autolyzing enzymes or with an additional small amount of peptidase agents, leading to economical manufacture.
3. Although culled chicken or chicken bone contains a large amount of muscular protein components, they have not been utilized as extract so far. Utilization of the muscular protein of culled chicken or chicken bone significantly elevates a nitrogen utilization level.

The extract obtained from chicken bone by hydrothermal extraction cannot be highly decomposed with enzymes. Therefore, muscular protein from chicken bone was solubilized with a weakly alkaline solution, heated to extract total protein, and decomposed with enzymes in a similar manner. As a result, an increase in the amination ratio was observed. In other words, it was confirmed that as the ratio of gelatin in the protein decreased the amination ratio increased, which shows that the gelatin component, which is solubilized structural protein, hinders decomposition. Consequently, it was confirmed that decomposition was efficiently accelerated by decomposing muscular protein alone free from dissolution of structural protein.

With regard to chicken bone, the non-patent document 1 describes that chicken bone includes protein content in a range of 15-22%, which is at the same level as that of ordinary meat, and can be utilized as a raw material for obtaining protein (See Table 3: General Components of Animal Bone). However, in this non-patent document 1, it is also described that protein is fractionated into water-soluble protein, salt-soluble protein, and insoluble protein; where 55-65% of total protein is insoluble protein, and digestibility and nutrition values should be evaluated. Bone of beef and pork contains a large amount insoluble protein, whereas chicken bone containing less insoluble protein is thus useful as a raw material.

[Table 3]

| Analyzed items | | Beef bone | Pork bone | Chicken bone | Pork shoulder |
|---|---|---|---|---|---|
| Water | % | 38.8 | 30.3 | 63.2 | 66.2 |
| Protein | % | 19.7 | 22.3 | 15.6 | 17.5 |
| Fat | % | 18.1 | 21.6 | 15.4 | 15.1 |
| Ash | % | 23.8 | 25.8 | 5.6 | 0.9 |
| Calcium | mg% | 7800 | 5600 | 1900 | 5 |
| Phosphorous | mg% | 4400 | 2570 | 996 | 160 |
| Magnesium | mg% | 159 | 114 | 46 | - |
| Iron | mg% | 8.6 | 5.5 | 5.1 | 1.3 |

As an extracting method for muscular protein, methods of treating with a neutral salt solution or a diluted alkaline solution are known. An attempt to utilize muscular protein in chicken bone has already been made in the U. S. since 1970's. There is a report that muscular protein was extracted with a neutral salt solution or a diluted alkaline solution and pulverized for utilization as a meat extender (See the non-patent document 2, for example). Furthermore, a technique for utilizing muscular protein from bone or the like as a seasoning is disclosed in the patent document 1, in which bone is decomposed with rice malt while decomposition of protein has not been taken into account, leaving the technique only for a flavoring agent due to its lower amination ratio.

In order to extract muscular protein alone, water, a weakly alkaline solution or enzymes is reacted at a temperature at which structural protein is not dissolved. By extracting protein at a temperature at which structural protein (mainly, collagen) is not dissolved, namely, without heating, autolyzing enzymes can be extracted together. In addition, the extracted autolyzing enzymes were found to contain a large amount of enzymes having a strong peptidase effect.

A large amount of peptidase which is required for decomposition at a high level is contained in bone. Chicken bone has more muscular protein and more autolyzing enzymes compared with pork bone or beef bone and thus is considered to be very suitable for a raw material. By utilizing autolyzing enzymes contained in a raw material, decomposition at a high level can be realized without adding a peptidase agent at all or with only a small amount of additional enzymes. In other words, extraction of muscular protein alone from chicken bone and utilization of autolyzing enzymes thereof enable practical and economical manufacture of seasonings from chicken prepared by decomposition with enzymes.

Based on the above-described effect, in the process for producing a protein hydrolysate according to the present invention, when decomposing protein of meat or a bone residue after taking meat (deboned meat), especially chicken meat, culled chicken or chicken bone, the protein in a state containing muscular protein alone is decomposed only with autolyzing enzymes or with autolyzing enzymes together with an enzymatic agent containing peptidase. By extracting muscular protein alone and utilizing autolyzing enzymes contained therein, the protein can be highly decomposed only with the autolyzing enzymes or with an additional small amount of an enzymatic agent having a peptidase effect.

Fig. 1 shows a result of extracting salt soluble protein (muscular protein) alone and decomposing it with a peptidase agent added thereto. In Fig. 1, an abscissa indicates time and an ordinate indicates amination ratios. According to Fig. 1, even a sole use of an autolyzing enzyme exhibited decomposition at a high level. In addition, the decomposition ratio increased in proportion to an amount of peptidase added. All of the lines (a), (b) and (c) in Fig. 1 showed the decomposition ratios of 55% or more amination ratios, and glutamic acid (Gul) / total nitrogen (T-N) was 0.7. In a sensory test, no bitterness was sensed and the taste was balanced, which was not inferior to HAP and HVP. Incidentally, effective ingredients such as taurine, creatine, anserine and carnosine contained in meat remained intact and were condensed as they were, which is deemed to be effective for health in addition to the good taste.

Collagen (or gelatin), which is a main component of structural protein, has a specific amino acid composition, with its characteristic of a large amount of oxyproline content which is not contained in muscular protein (see Table 4: Amino acid compositions of main proteins in meat, Amano et al., "Handbook for Food Processing").

[Table 4]

| | Muscular protein | | | Structural protein | |
|---|---|---|---|---|---|
| | Myosin | Actin | Myoglobin | Collagen | Elastin |
| Isoleucine | 5.5 | 8.5 | 16.8 | 1.9 | 3.8 |
| Leucine | 10.4 | 8.5 | | 3.7 | 9.0 |
| Lysine | 12.4 | 6.7 | 15.5 | 4.0 | 0.5 |
| Methionine | 3.3 | 5.2 | 1.7 | 1.0 | - |
| Phenylalanine | 4.5 | 4.6 | 5.1 | 2.3 | 6.2 |
| Threonine | 4.9 | 6.9 | 4.6 | 2.3 | 1.1 |
| Tryptophan | - | 2.0 | 2.3 | - | - |
| Valine | 4.9 | 5.6 | 4.1 | 2.5 | 17.7 |
| Arginine | 7.1 | 7.0 | 2.2 | 8.2 | 1.3 |
| Histidine | 2.5 | 2.9 | 8.5 | 0.7 | - |
| Alanine | 6.9 | 6.4 | 8.0 | 10.3 | 21.3 |
| Aspartic acid | 11.3 | 10.9 | 8.2 | 6.9 | 1.1 |
| Cystine | 1.9 | 1.9 | - | - | 0.4 |
| Glutamic acid | 22.8 | 14.1 | 16.5 | 11.2 | 2.4 |
| Glycine | 2.9 | 5.2 | 5.9 | 26.6 | 26.7 |
| Proline | 2.5 | 5.1 | 3.3 | 14.4 | 13.5 |
| Serine | 4.3 | 5.3 | 3.5 | 4.3 | 0.9 |
| Tyrosine | 3.3 | 6.5 | 2.4 | 1.0 | 1.5 |
| Oxyproline | - | - | - | 12.8 | 1.6 |

The above values indicate gram units per 100 grams of protein.

According to the process of producing a protein hydrolysate in the present invention, protein can be decomposed at a high level (amination ratio: 55% or more) by utilizing muscular protein alone excluding structural protein. In addition, the protein hydrolysate obtained by this method (final product) contains decomposed muscular protein as well as trace constituents included in muscular protein such as taurine, anserine, carnosine and creatine. Namely, according to this process, muscular protein alone is selectively decomposed and thus does not contain oxyproline while the above trace constituents which are usually included in muscular protein remain contained.

In the case that chicken meat is used as a raw material, trace constituents in meat (taurin, anserine, carnosine, creatine, etc.) are contained while oxyproline in protein is controlled to be not more than 1.0% by mass. In other words, oxyproline in protein of general chicken (breast meat) is 0.8% by mass (known from Table 2), but oxyproline in a seasoning prepared by decomposing muscular protein alone is controlled to be 0.5% by mass or less. In the case of chicken bone or culled chicken, oxyproline in protein of chicken bone is approximately 4-5% by mass (known from Table 2 and the non-patent document 1), while in the present process in which structural protein is removed before decomposition, the amount is controlled to be 1.0% by mass or less.

Preferably, muscular protein is extracted from chicken meat, culled chicken or chicken bone by reacting water, a weakly alkaline solution, or enzymes at a temperature at which structural protein is not dissolved. By extracting protein at a temperature at which structural protein is not dissolved, or a temperature at which collagen in structural protein is not gelatinized, an extract solution which comprises muscular protein alone and does not contain gelatin can be obtained. Furthermore, since autolyzing enzymes are not impaired at a temperature at which structural protein is not dissolved, the extracted muscular protein can be highly decomposed with the autolyzing enzymes.

Preferably, the extraction of muscular protein is carried out at 60°C or below. At a temperature of 60°C or below, structural protein is not dissolved, and it is less likely to occur that collagen in structural protein is gelatinized and eluted. Furthermore, autolyzing enzymes are not impaired. Extraction above 60°C causes dissolution of structural protein and damage on autolyzing enzymes, which leads to lower efficiency in decomposing muscular protein.

In the process of producing a protein hydrolysate according to the present invention, residues of meat after thermal extraction are mixed with the above-described muscular protein, especially muscular protein extracted from chicken bone containing autolyzing enzymes, and decomposed utilizing the autolyzing enzymes only or by the autolyzing enzymes along with an effect of an enzymatic agent containing peptidase. The residues after thermal extraction do not contain structural protein, but the autolyzing enzymes are impaired by heating. By mixing the residues with muscular protein in a state of muscular protein alone as described above, the residues can be highly decomposed with only the autolyzing enzymes contained in the muscular protein or by the autolyzing enzymes along with an effect of an enzymatic agent containing peptidase.

Here, preferably, the residues are solubilized with an enzymatic agent containing endoprotease before mixing with the muscular protein. The residues are not limited to chicken meat, but can be any meat such as beef and pork after thermal extraction. Protease is classified into proteinase (endopeptidase = endo-type) and peptitdase (exopeptidase = exo-type). Proteinase roughly cuts protein to form peptone and peptide, and peptidase cuts peptide one by one from the end to form amino acids. Amination ratios are not elevated in the endo-type but increased only when using the exo-type. In general, protease means the endo-type. The reason for solubilizing the residues with endoprotease is to emphasize the following peptidase effect. Although it is possible to use the endo-type mixed with the exo-type, analysis of the following peptidase effect will be difficult then.

## ADVANTAGE OF THE INVENTION

Since the process for producing a protein hydrolysate of the present invention employs chicken meat, chicken bone, or culled chicken, which has less structural protein compared to beef or pork, muscular protein can be efficiently decomposed.

Furthermore, utilization of autolyzing enzymes enables decomposition of protein at a high level with improved efficiency, thereby producing animal protein hydrolysates (seasonings prepared by enzymatic decomposition) economically.

In addition, by heating solid residues after extracting muscular protein with water, gelatin extract can also be obtained, resulting in a higher nitrogen utilization ratio.

Compared to conventional thermal extraction methods, the present invention enhances a nitrogen utilization ratio and, furthermore, realizes the manufacture of safer seasonings prepared by enzymatic decomposition.

## BRIEF EXPLANATION OF THE DRAWINGS

Fig. 1 is a graph showing transition of amination ratios in enzymatic decomposition.

Fig. 2 is a graph showing transition of decomposition ratios using commercially-available peptidase agents.

Fig. 3 is a graph showing transition of amination ratios in decomposition of mixture of meat and a muscular protein extract solution of chicken bone.

Fig. 4 is a flow chart summarizing a process for producing a protein hydrolysate in embodiments of the present invention.

## BEST MODE FOR CARRYING OUT THE INVENTION

Best modes for carrying out a process for producing a protein hydrolysate according to the present invention will be explained below.

(A) Production of seasonings prepared by enzymatic decomposition from chicken meat, culled chicken, and chicken bone

(1) Raw material

Examples of a raw material which can be used are chicken meat, carcasses (bodies without heads, wings, organs and legs) of culled chickens (adult chickens after egg laying), carcasses of broilers, bone remaining after taking chicken meat (chicken bone), or the like.

To obtain seasonings prepared by enzymatic decomposition as protein hydrolysates, muscular protein is used in principle. Therefore, a material containing a large amount of meat portion is preferable. In this respect, carcasses of culled chickens which have tough meat and are almost useless and chicken bone which is by-products of chicken meat production is most suitable because they are stably available in large quantity and are inexpensive.

In addition to the above, scrap meat with a low utility value can be used as a raw material.

(2) Pretreatment of raw material

A raw material is minced beforehand for extracting muscular protein with water. An appropriate mincing size is 3-10mm.

In the pretreatment process, it is possible to remove tendons, skins, bone, and the like which contain structural protein. Namely, meat, bone or the like is finely chopped and sieved through fine screens, thereby removing structural protein.

(3) Extraction of muscular protein and separation of solid matter

100-300 parts by mass of water is added to 100 parts by mass of a minced (shredded) material and stirred.

To extract muscular protein, some amount of alkali or nothing is added thereto, or an enzyme for protein decomposition is added to dissolve protein.

When muscular protein is extracted by water or weak alkali, the extracted solution has viscosity. Therefore, some treatment with enzyme is beneficial in decreasing viscosity.

Here, it should be noted that enzymes and extraction conditions are selected so as not to exceed the temperature at which collagen is gelatinized and not to impair autolyzing enzymes while keeping a high protein recovery rate.

Enzymes for decomposing protein are not specifically limited here. For example, the following enzymes can be used:

(a) Amano Pharmaceutical Co., Ltd.: Protease N, S (trademark)
(b) Novo Nordisk Pharma Ltd.: Alkarase (trademark), Protamex (trademark)
(c) Daiwa Kasei K. K.: Protin PC (trademark)
(d) HBI Enzymes Inc. (Hankyu Kyoei Bussan Co., Ltd.): Orientase 22BF, 90N (trademark)

When a weakly alkaline solution is added, pH adjustment is necessary before adding an enzyme.

An appropriate pH range is 6-8 (optimum pH) at which autolyzing enzymes easily work.

A preferable temperature is 40-55°C where collagen is not gelatinized, and a preferable duration is 0.5-2 hours taking into account the relation of collagen and bacteria growth (prevention of decay). Usually, salt is added to prevent decay during decomposition. However, in the producing process of the present embodiment, as the decomposition is completed within a short time, it is not necessary to add salt during decomposition.

An amount of an enzyme to be added for protein decomposition depends on an amount of protein in the material, but is preferably in a range from 0.05-2.0% to 100 parts by mass of a raw material.

When an enzymatic action has progressed and muscular protein is dissolved, insoluble matter are removed with a wire mesh of 30-60 mesh to separate a solution part in which muscular protein have been dissolved from insoluble solid matter.

The solid part, which contains a large amount of collagen, can be utilized as a material for a gelatin-like extract by thermal extraction.

(4) Decomposition of muscular protein

The extracted muscular protein solution, which contains oil and fat contents, is centrifuged to separate the oil and fat contents.

The extracted solution contains autolyzing enzymes and the added enzymes. The remaining activities of these enzymes are different depending on the extraction conditions, and are decomposed as they are or, where necessary, by adding an enzymatic agent having a peptidase effect.

The kinds of the enzymes are not specifically limited. For example, the following enzymes can be used:

(a) Amano Pharmaceutical Co., Ltd.: Protease A, P, M (trademarks), Peptidase R (trademark)
(b) Novo Nordisk Pharma Ltd.: Flavorzyme (trademark)
(c) Crude enzymes extracted from rice malt

A preferable enzyme to be added is an enzyme with a strong peptidase activity, especially, an enzyme derived from rice malt mold *(Genus Aspergillus* (Asp.)).

Decomposition is carried out at a temperature of 40-60°C and completed in 2-40 hours, resulting in an amination ratio of 55% or more.

(5) Production of gelatin-like extract from separated solid matter

The solid matter remaining after separating muscular protein contains a large amount of collagen, from which a gelatin-like extract is obtained by heating with water.
100-300 part by mass of water is added to 100 parts by mass of solid matter and heated at 80-100°C for the duration from 30 minutes to 2 hours to obtain a gelatin-like extract. Insoluble matter and oil and fat contents are removed, and then purified and condensed in an ordinary manner to obtain a chicken extract.

(6) Preparation of hydrolyzed seasoning
After completion of the muscular protein decomposition, an amination ratio is confirmed, and the resultant is purified according to a conventional method. Namely, a seasoning is obtained by adding activated charcoal and heating, followed by discoloration and filtration, or by sterilizing the resultant by heating as it is.
Alternatively, the resultant is condensed, followed by sediment removal (precipitated sediment is eliminated) in the middle of the process, and then further condensed to obtain a seasoning.

(7) Protein recovery ratio
Table 5 shows protein recovery ratios of chicken bone in a thermal extraction method and in an improved method (the process of the present invention).
According to the process of the present invention, the protein recovery ratio is high, and an amount of residues is significantly reduced, which shows utility of the present invention.

[Table 5]

| | Thermal extraction method (conventional method) | Improved method (present invention) | |
|---|---|---|---|
| Raw material | Chicken bone | Chicken bone | |
| Extraction method | Thermal extraction<br>115°C<br>1 hour | (1) Extraction of meat part Enzymatic treatment<br>(2) Residue Thermal extraction | |
| Protein recovery ratio /raw material % | 5.0 | (1)<br>(2)<br>Total | 7.5<br>3.5<br>11.0 |
| Protein recovery ratio /raw material protein % | 32.7 | (1)<br>(2)<br>Total | 49.0<br>22.9<br>71.9 |
| Amount of residue /raw material % | 76.5 | | 33.3 |

(B) Production of seasonings prepared by enzymatic decomposition from residues after thermal extraction of chicken meat
An appropriate raw material for this invention is chicken breast meat containing less structural protein. A stable supply of chicken, especially chicken breast meat is available and thus can be used as a raw material for producing meat extract. Such chicken meat is minced, 2-3 times amount of water is added thereto, and the resultant is decocted for 2-5 hours to obtain an extract. The extract is either condensed or uncondensed for use as soup or broth.
A residue after the extraction serves as a raw material of a seasoning prepared by enzymatic decomposition produced in the present embodiment.
The residue after extraction is solubilized with endoprotease. Here, it is confirmed that no structural protein (gelatin component) is contained. Chicken meat residue after extraction has an inherently lower structural protein content. In addition, due to heating and extraction, its gelatin content is low. In the case that a proportion of gelatin component is high, the residue is further heated to remove the gelatin component.

The endoprotease agent to be used is not limited specifically. An agent that increases a dissolution ratio of protein as much as possible is preferable, and the following agents can be used, for example:

(a) Amano Pharmaceutical Co., Ltd.: Protease S, N (trademark)
(b) Novo Nordisk Pharma Ltd.: Alkarase (trademark), Protamex (trademark)
(c) Daiwa Kasei K. K.: Thermoase (trademark), Protin (trademark)
(d) HBI Enzymes Inc.: Orientase N, 22BF (trademark)

To the residue after extraction, 2-5 times amount of water is added and decomposed at optimum pH and temperature depending on the added enzyme until it shows a maximum dissolution ratio.
Here, 0.02-0.2kg of enzyme added to 10kg is sufficient.
The dissolution ratio can be represented by the soy sauce test method, in which the value was 80-90%. The amination ratio was 10-20%.
After completing solubilization, a solubilized solution from which insoluble matter is either removed or not is prepared. The solubilized solution contains almost no trace constituents such as taurin, anserine, carnosine and creatine derived from meat.
The chicken meat residue after extraction was solubilized as above, to which an enzymatic agent containing peptidase was reacted. Then, the highest amination ratio was 45-50%, and further decomposition was not shown. Even an increased amount of peptidase and extended reaction time did not improve the decomposition ratio. This means that the commercially-available enzymes had limitations in their decomposition ratios. The decomposition ratios differed depending on the enzymes used (see Fig. 2). In many cases, the limited values are caused by product inhibition due to accumulation of generated amino acids. However, in a test where a large amount of purified amino acid was added during decomposition, inhibition by the added amino acid was hardly observed. In view of this, the present inventors deemed that the reason for the limited decomposition ratios was that there was a part that the used enzymes could not further decompose and the decomposition process was stopped.
Therefore, in the process of the present invention, the extracted solution of muscular protein of chicken bone specified in the above (A) is mixed with the solubilized solution before decomposition. By decomposing the solubilized solution mixed with the extracted solution containing autolyzing enzymes of chicken bone, the decomposition ratio has no limitation, enabling decomposition at a high level. Fig. 3 is a graph showing transition of amination ratios in this case. An autolyzing enzyme in chicken bone is suitable for high decomposition of muscular protein. Without an autolyzing enzyme after heating, an additional autolyzing enzyme from other materials enables decomposition at a high level.
In this case, addition of an enzymatic agent containing peptidase accelerates the decomposition process. The peptidase agent is not specifically limited. For example, the following agents can be used:

(a) Amano Pharmaceutical Co., Ltd.: Protease A, P, M, Peptidase R (trademark)
(b) Novo Nordisk Pharma Ltd.: Flavorzyme (trademark)
(c) Crude enzymes extracted from rice malt

As an enzyme to be added, the one having a strong peptidase activity, especially derived from *Aspergillus* is preferable.
A temperature for decomposition is 40-60°C, and the decomposition is completed in 5-40 hours, resulting in an amination ratio of 55% or more.
After the above process, the decomposed matter is adjusted in a manner similar to the above (A) to obtain a seasoning.
As described above, in the process for producing a protein hydrolysate in the present embodiment, muscular protein extracted from chicken bone (containing autolyzing enzymes) is decomposed either as it is or after mixing with other muscular protein solution so that high decomposition is realized and that an animal-derived seasoning prepared by enzymatic decomposition is obtained. In addition, an extracted solution of muscular protein from chicken bone is utilized as a peptidase agent, enabling decomposition at a high level and a low cost. Moreover, since enzyme components are condensed for use, enzymes can be recovered from the solution after decomposition and reused.
Autolyzing enzymes exist not only in chicken bone but also in pork or beef bone, which can be utilized as well. However, while chicken bone (or culled chicken) is soft enough to be easily minced, pork and beef bone is hard and must be finely ground. In this case, fine grinding with a millstone or the like leads to deactivation of enzymes due to heat generation, which should be prevented. Therefore, the operation becomes complicated compared to mincing. In this respect, chicken bone (or culled chicken) is appropriate because they are soft and easily minced without generating heat.
Only muscular protein is extracted from chicken bone and decomposed to obtain a decomposed seasoning while a gelatin-like extract can be obtained by heating residues remaining after extraction which contain collagen as a main component. In other words, muscular protein which has not been utilized in thermal extraction heretofore is used, thereby considerably increasing utilization ratio of protein and reducing residues at a significant level.

Fig. 4 is a flow chart summarizing the process for producing a protein hydrolysate in the present embodiment.
Next, specific examples of the present invention will be explained below.

Example 1

(Seasoning prepared by enzymatic decomposition produced from chicken meat)
25kg of water was added to 10kg of minced chicken meat and heated while stirring up to 45°C. 10g of protein decomposing enzyme (Protease N (Amano Pharmaceutical Co., Ltd.)) was added thereto and stirred for one hour. Then, the resultant mixture was filtrated with a wire mesh of 30 mesh to separate solid matter.
The solution after filtration was centrifuged to remove oil and fat contents. With additional 50g of enzyme (Protease P (Amano Pharmaceutical Co., Ltd.)) to 30.7kg of the resultant solution, the solution was decomposed at 55°C for 15 hours. After decomposition, the enzyme was deactivated by heating at 90°C for 30 minutes, followed by removing insoluble matter to obtain a transparent solution.
The obtained solution was condensed to have 35% solid contents with a vacuum condenser, and deposited precipitation was removed, followed by further condensation. As a result, 2.6kg of seasoning prepared by enzymatic decomposition having 70% solid contents was obtained.
Then, 5kg of water was added to 2.6kg of solid matter separated with a wire mesh and heated at 90°C for 30 minutes. After removing insoluble matter and oil and fat contents, the solution was condensed to obtain 0.2kg of chicken extract having 60% solid contents.
The resultant seasoning prepared by enzymatic decomposition was highly decomposed because of elimination of structural protein and action of autolyzing enzymes and the peptidase agent.
The analytical values of the obtained seasonings prepared by enzymatic decomposition are shown in Table 6. As shown in the values in Table 6, the obtained seasoning prepared by enzymatic decomposition contains trace constituents of meat such as taurin, anserine, carnosine and creatine, and oxyproline (Hyp)/protein (C-P) is 0.06% meeting the requirement that oxyproline is 0.5% by mass or less to protein.
[Table 6]

| | | Example 1 Chicken meat | Example 2 Chicken bone | Example 3 Culled chicken | Example 4 Chicken bone + residue of chicken meat after extraction |
|---|---|---|---|---|---|
| | | Decomposed seasoning | Decomposed seasoning | Decomposed seasoning | Decomposed seasoning |
| Total nitrogen (T-N) | % | 7.49 | 7.48 | 7.35 | 7.48 |
| Formole nitrogen (F-N) | % | 4.12 | 4.21 | 4.39 | 4.20 |
| Glutamic acid (Glu) | % | 4.48 | 5.42 | 5.46 | 4.78 |
| Oxyproline (Hyp) | % | 0.027 | 0.081 | 0.103 | 0.18 |
| Total creatine | % | 1.08 | 0.83 | 0.74 | 0.55 |
| Taurin | % | 0.25 | 0.506 | 0.297 | 0.29 |
| Carnosine | % | 0.304 | 0.571 | 0.348 | 0.36 |
| Anserine | % | 0.583 | 0.444 | 0.336 | 0.34 |
| Amination ratio | % | 55.0 | 56.2 | 59.7 | 56.1 |
| Glu/T-N | % | 0.60 | 0.72 | 0.74 | 0.64 |
| Hyp/C-P | % | 0.06 | 0.17 | 0.22 | 3.8 |

Method of Analysis

T-N: Soy sauce test method
F-N: ditto
Glu: Yamasa L-glutamin measuring kit
Hyp: decomposition-colorimetiric assay method
Total creatine: alkaline picric acid method (Jaffe method)
Taurin: high-performance liquid chromatography (HPLC method)
Carnosine: ditto
Anserine: ditto

Example 2

(Seasoning prepared by enzymatic decomposition produced from chicken bone)
20kg of water was added to 10kg of minced chicken bone and heated with stirring to 45°C.
32g of enzyme (Alkarase (Novo Nordisk Pharma Ltd.)) was added and stirred for one hour. Then, the resultant mixture was filtrated with a wire mesh of 30 mesh to separate solid matter.
The solution after filtration was centrifuged to remove oil and fat contents and decomposed with stirring at 50°C for 12 hours.
After heating at 90°C for 30 minutes, insoluble matter was removed, and the solution was condensed to have 35% solid contents. Deposited precipitation was filtrated, followed by further condensation, to obtain 1.3kg of seasoning prepared by enzymatic decomposition having 70% solid contents.
10.5kg of water was added to 5.2kg of solid matter separated with a wire mesh and heated at 90°C for 30 minutes.
After removing insoluble matter and oil and fat contents, the solution was condensed to obtain 0.76kg of chicken extract having 60% solid contents.
The resultant seasonings was highly decomposed only with an action of autolyzing enzymes. As shown in the analytical values in Table 6, the seasoning prepared by enzymatic decomposition contains trace constituents of meat such as taurin, anserine, carnosine and creatine, and Hyp/C-P is 0.17% meeting the requirement that oxyproline is 1% by mass or less to protein.

Example 3

(Seasoning prepared by enzymatic decomposition produced from culled chicken)
20kg of water was added to 10kg of minced culled chicken (carcasses without organs) and heated with stirring up to 45°C. 10g of enzyme (Orientase 90N (HBI Enzymes Inc.)) was added thereto and stirred for 2 hours. The resultant mixture was filtrated with a wire mesh of 30 mesh.
The solution after filtration was centrifuged to remove oil and fat contents. With additional 25g of enzyme (Flavorzyme (Novo Nordisk Pharma Ltd.)) to 21.5kg of the resultant solution, the solution was decomposed at 50°C for 15 hours.
After decomposition, the solution was heated at 90°C for 30 minutes, followed by removing insoluble matter to obtain 21.0kg of transparent solution.
The obtained solution was condensed to have 35% solid content, and deposited precipitation was removed, followed by further condensation. As a result, 1.38kg of seasoning prepared by enzymatic decomposition having 70% solid content was obtained.
10.4kg of water was added to 5.2kg of solid matter separated from the wire mesh and heated at 90°C for 30 minutes.
After removing insoluble matter and oil and fat contents, the resultant solution was condensed to obtain 0.62kg of chicken extract having 60% solid content.
The obtained seasoning was highly decomposed with autolyzing enzymes and the added enzymes.
As shown in the analytical values in Table 6, the seasoning prepared by enzymatic decomposition contains trace constituents of meat such as taurin, anserine, carnosine and creatine, and Hpy/C-P is 0.22% meeting the requirement that oxyproline is 1% by mass or less to protein.

Example 4

(Seasoning prepared by enzymatic decomposition produced from residue of chicken meat after extraction)
In the case of chicken breast meat , water was added to the meat and subject to extraction with heating for 1-5hours.
The obtained extract was used as bouillon or sung-tong in cooking. The remaining residue was utilized as a raw material of this example.
(A) Solubilization of meat residue after extraction

20kg of water was added to 5kg of meat residue, and 0.05kg of Protease N (Amano Pharmaceutical Co., Ltd.) was added thereto. The resultant mixture was solubilized with stirring at 60°C for 15 hours.
After removing insoluble matter, 21.3kg of solubilized solution (A) was obtained.
Then, T-N was 0.68%, and the amination ratio was 14%.

(B). Decomposition of solubilized solution
20kg of water was added to 10kg of minced chicken bone. With 10g of Orientase 22BF (HBI Enzymes Inc.) added thereto, the mixture was decomposed at 45°C for one hour. After removing oil and insoluble matter, 21.3kg of meat extracted solution (B) of chicken bone was obtained.
The solubilized solution (A) of chicken meat residue after extraction was mixed with the extracted solution (B) of chicken bone. 70g of Flavorzime 1000L (Novo Nordisk Pharma Ltd.) was added thereto and decomposed at 50°C for 30 minutes. After confirming 55% or more of amination ratio, the resultant mixture was heated at 90°C for 30 minutes, insoluble matter was removed, followed by condensation to have 35% solid matter, and deposited precipitation was filtrated. After further condensation, 3.0kg of seasoning prepared by enzymatic decomposition having 60% solid content was obtained. The analytical values are shown in Table 6.
While examples of the present invention have been described above, it is to be understood that the invention is not limited to the above examples and that various changes and modifications may be made without departing from the gist of the invention.

INDUSTRIAL APPLICABILITY

The process for producing a protein hydrolysate of the present invention is useful as a process for obtaining a protein hydrolysate by hydrolyzing with enzymes protein of meat or a bone residue remaining after taking meat, especially chicken meat, chicken bone or culled chicken. In particular, the process is preferable for decomposition at an amination ratio of 55% or more and for obtaining a highly safe protein hydrolysate at the same time.

**Claims**

1. A process for producing a protein hydrolysate comprising, when decomposing protein of meat or a bone residue after taking meat, decomposing the protein in a state containing muscular protein alone utilizing an autolyzing enzyme only or by an action of the autolyzing enzyme with an enzymatic agent containing peptidase.

2. The process for producing a protein hydrolysate as claimed in claim 1, wherein said muscular protein is extracted from the meat or the bone residue by reacting water, a weakly alkaline solution, or an enzyme at a temperature at which structural protein is not dissolved.

3. The process for producing a protein hydrolysate as claimed in claim 2, wherein said muscular protein is extracted at a temperature of 60°C or below.

4. The process for producing a protein hydrolysate as claimed in claim 1, wherein said meat is chicken meat or culled chicken, and said bone residue is chicken bone.

5. The process for producing a protein hydrolysate as claimed in claim 2, wherein said meat is chicken meat or culled chicken, and said bone residue is chicken bone.

6. The process for producing a protein hydrolysate as claimed in claim 3, wherein said meat is chicken meat or culled chicken, and said bone residue is chicken bone.

7. The process for producing a protein hydrolysate as claimed in claim 1, wherein, when chicken meat is used as a raw material, trace constituents in meat such as taurin, anserine, carnosine and creatine are contained with Hyp (oxyproline) being 0.5% by mass or less to protein, and when culled chicken or chicken bone is used as a raw material, trace constituents in meat such as taurin, anserine, carnosine and creatine are contained with Hyp (oxyproline) being 1.0% by mass or less to protein.

8. The process for producing a protein hydrolysate as claimed in claim 1, wherein a residue of meat after thermal extraction is mixed with said muscular protein and decomposed utilizing the autolyzing enzyme only or by the action of the autolyzing enzyme with the enzymatic agent containing peptidase.

9. The process for producing a protein hydrolysate as claimed in claim 2, wherein a residue of meat after thermal extraction is mixed with said muscular protein and decomposed utilizing the autolyzing enzyme only or by the action of the autolyzing enzyme with the enzymatic agent containing peptidase.

10. The process for producing a protein hydrolysate as claimed in claim 3, wherein a residue of meat after thermal extraction is mixed with said muscular protein and decomposed utilizing the autolyzing enzyme only or by the action of the autolyzing enzyme with the enzymatic agent containing peptidase.

11. The process for producing a protein hydrolysate as claimed in claim 4, wherein a residue of meat after thermal extraction is mixed with said muscular protein and decomposed utilizing the autolyzing enzyme only or by the action of the autolyzing enzyme with the enzymatic agent containing peptidase.

12. The process for producing a protein hydrolysate as claimed in claim 5, wherein a residue of meat after thermal extraction is mixed with said muscular protein and decomposed utilizing the autolyzing enzyme only or by the action of the autolyzing enzyme with the enzymatic agent containing peptidase.

13. The process for producing a protein hydrolysate as claimed in claim 6, wherein a residue of meat after thermal extraction is mixed with said muscular protein and decomposed utilizing the autolyzing enzyme only or by the action of the autolyzing enzyme with the enzymatic agent containing peptidase.

14. The process for producing a protein hydrolysate as claimed in claim 8, wherein said residue is solubilized with an enzymatic agent containing an endoproteasae and mixed with the muscular protein.

15. The process for producing a protein hydrolysate as claimed in claim 9, wherein said residue is solubilized with an enzymatic agent containing an endoproteasae and mixed with the muscular protein.

16. The process for producing a protein hydrolysate as claimed in claim 10, wherein said residue is solubilized with an enzymatic agent containing an endoproteasae and mixed with the muscular protein.

17. The process for producing a protein hydrolysate as claimed in claim 11, wherein said residue is solubilized with an enzymatic agent containing an endoproteasae and mixed with the muscular protein.

18. The process for producing a protein hydrolysate as claimed in claim 12, wherein said residue is solubilized with an enzymatic agent containing an endoproteasae and mixed with the muscular protein.

19. The process for producing a protein hydrolysate as claimed in claim 13, wherein said residue is solubilized with an enzymatic agent containing an endoproteasae and mixed with the muscular protein.

20. A protein hydrolysate produced by decomposing protein of meat or a bone residue after taking meat in a state containing muscular protein alone utilizing an autolyzing enzyme only or by an action of the autolyzing enzyme with an enzymatic agent containing peptidase.

21. The protein hydrolysate claimed in claim 20, wherein said meat is chicken meat or culled chicken, and said bone residue is chicken bone.

22. The protein hydrolysate claimed in claim 20, wherein, when chicken meat is used as a raw material, trace constituents in meat such as taurin, anserine, carnosine and creatine are contained with Hyp (oxyproline) being 0.5% by mass or less to protein, and when culled chicken or chicken bone is used as a raw material, trace constituents in meat such as taurin, anserine, carnosine and creatine are contained with Hyp (oxyproline) being 1.0% by mass or less to protein.

23. The protein hydrolysate as claimed in claim 20, wherein a residue of meat after thermal extraction is mixed with said muscular protein and decomposed utilizing the autolyzing enzyme only or by the action of the autolyzing enzyme with the enzymatic agent containing peptidase.

24. The protein hydrolysate as claimed in claim 21, wherein a residue of meat after thermal extraction is mixed with said muscular protein and decomposed utilizing the autolyzing enzyme only or by the action of the autolyzing enzyme with the enzymatic agent containing peptidase.

# FIG. 1

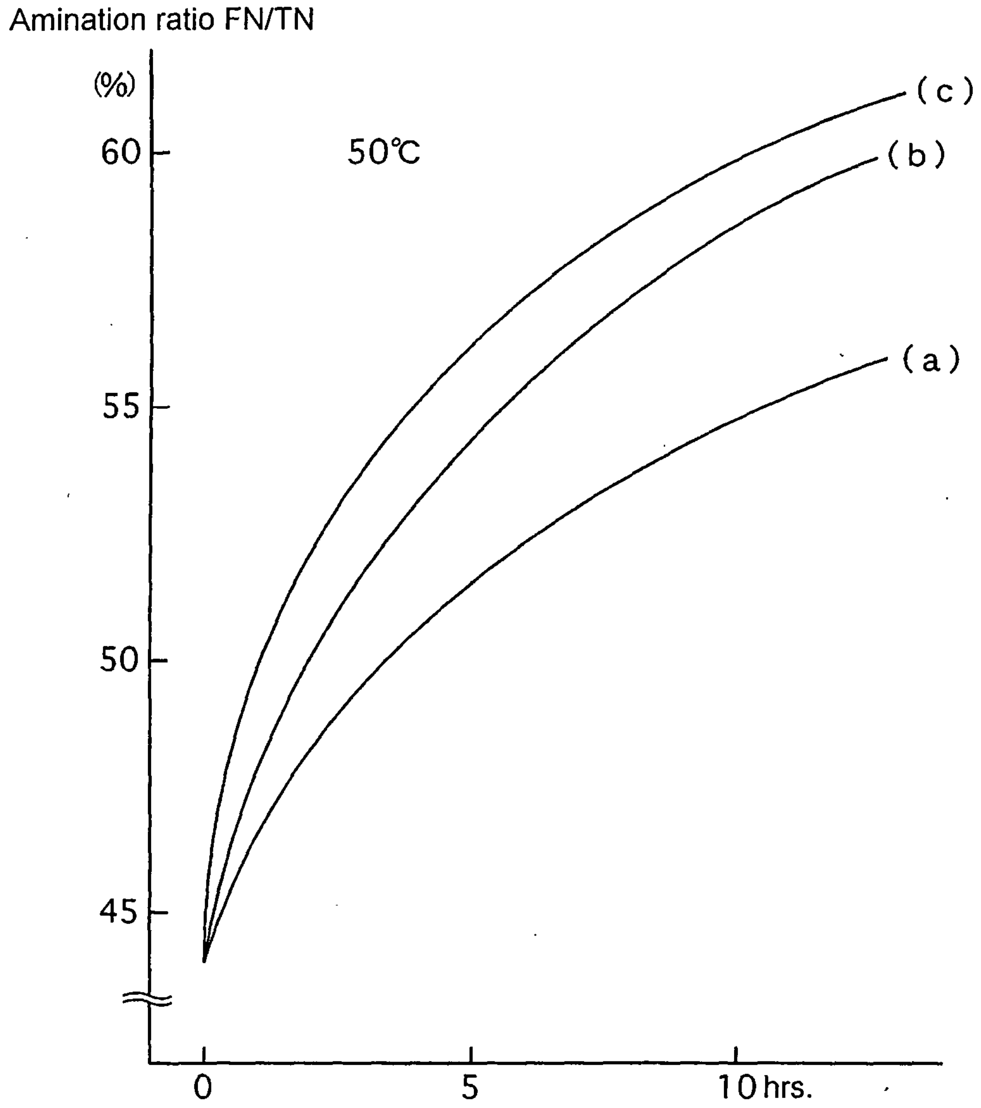


Raw material: chicken bone
muscular protein solution    concentration 3.7%
amination ratio 44.2%

Amount of added peptidase
(a) 0
(b) 2%/C-P
(c) 3%/C-P

The amination ratio upon extraction of muscular protein was 44%, which was the value of decomposition by autolyzing enzyme

# FIG. 2

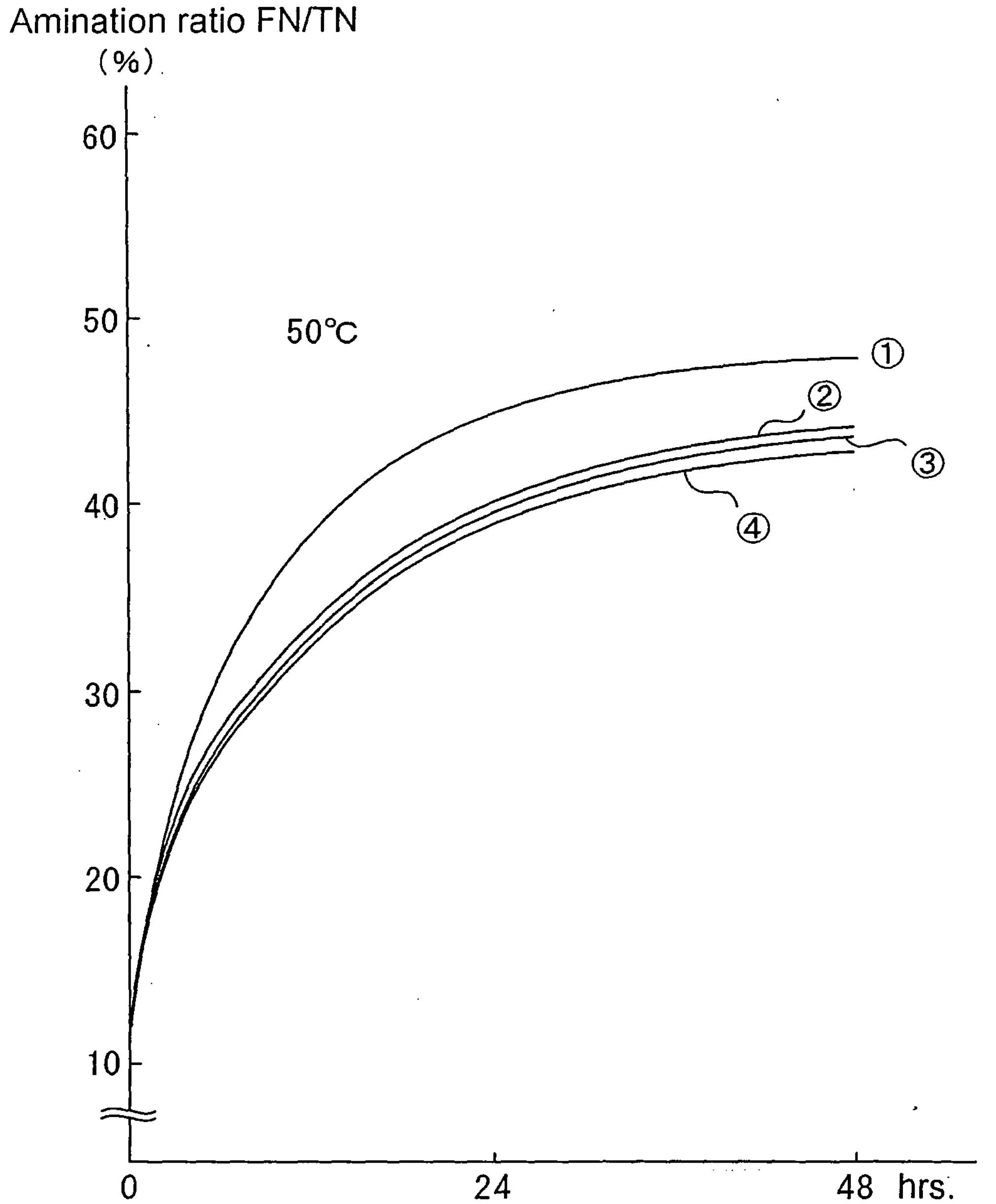


Transition of decomposition ratio by commercially-available peptidase agent

| | enzyme /muscular protein (%) | pH |
|---|---|---|
| ①. *Aspergillus oryzae* | 5.0 | 6.5 |
| ②. *Rhizopus oryzae* | 5.0 | 6.5 |
| ③. *Aspergillus oryzae* | 5.0 | 5.0 |
| ④. *Aspergillus melleus* | 5.0 | 6.5 |

FIG. 3

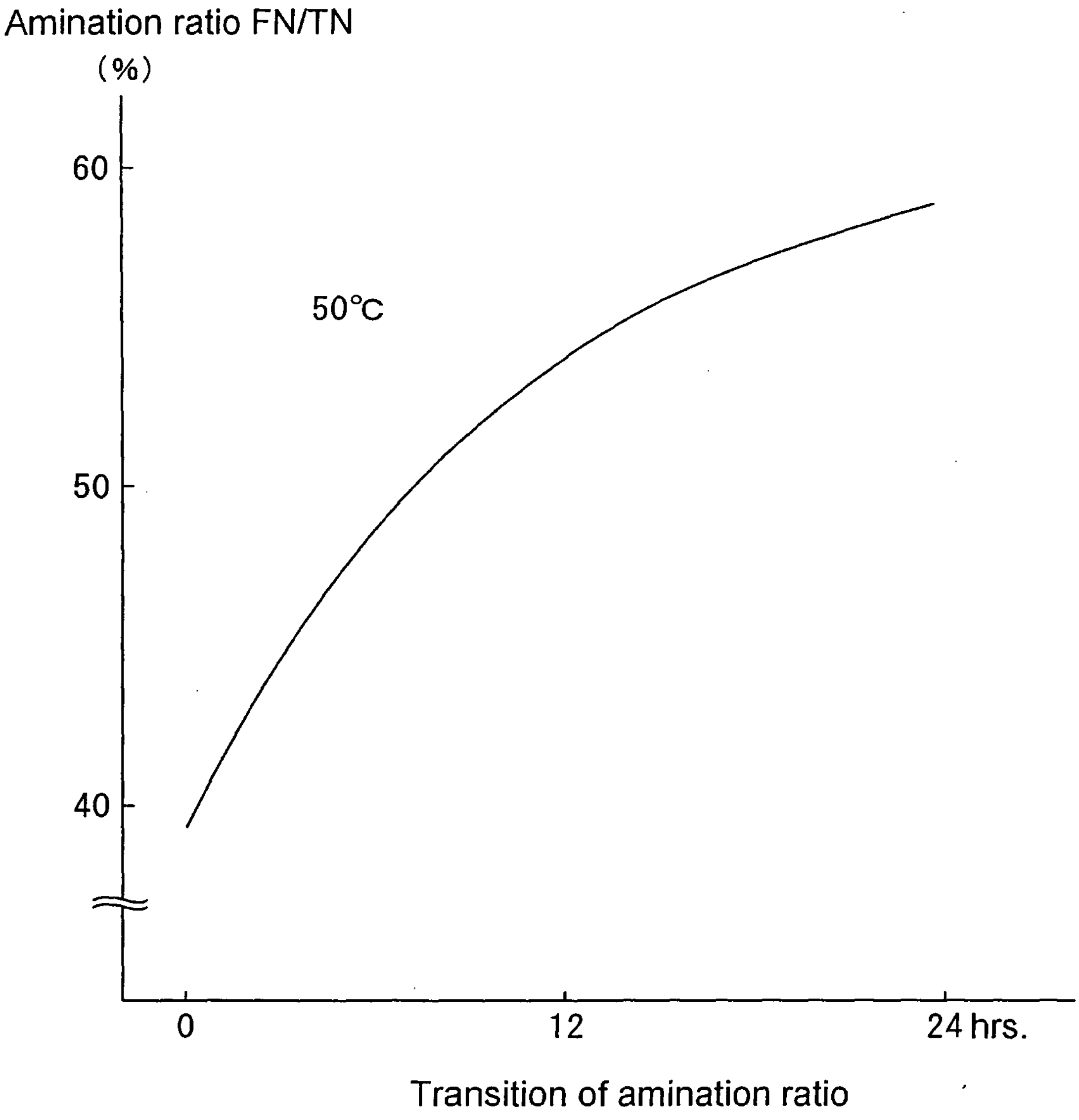
Amination ratio FN/TN
(%)
60
50
40
50°C
0
12
24 hrs.
Transition of amination ratio

FIG. 4

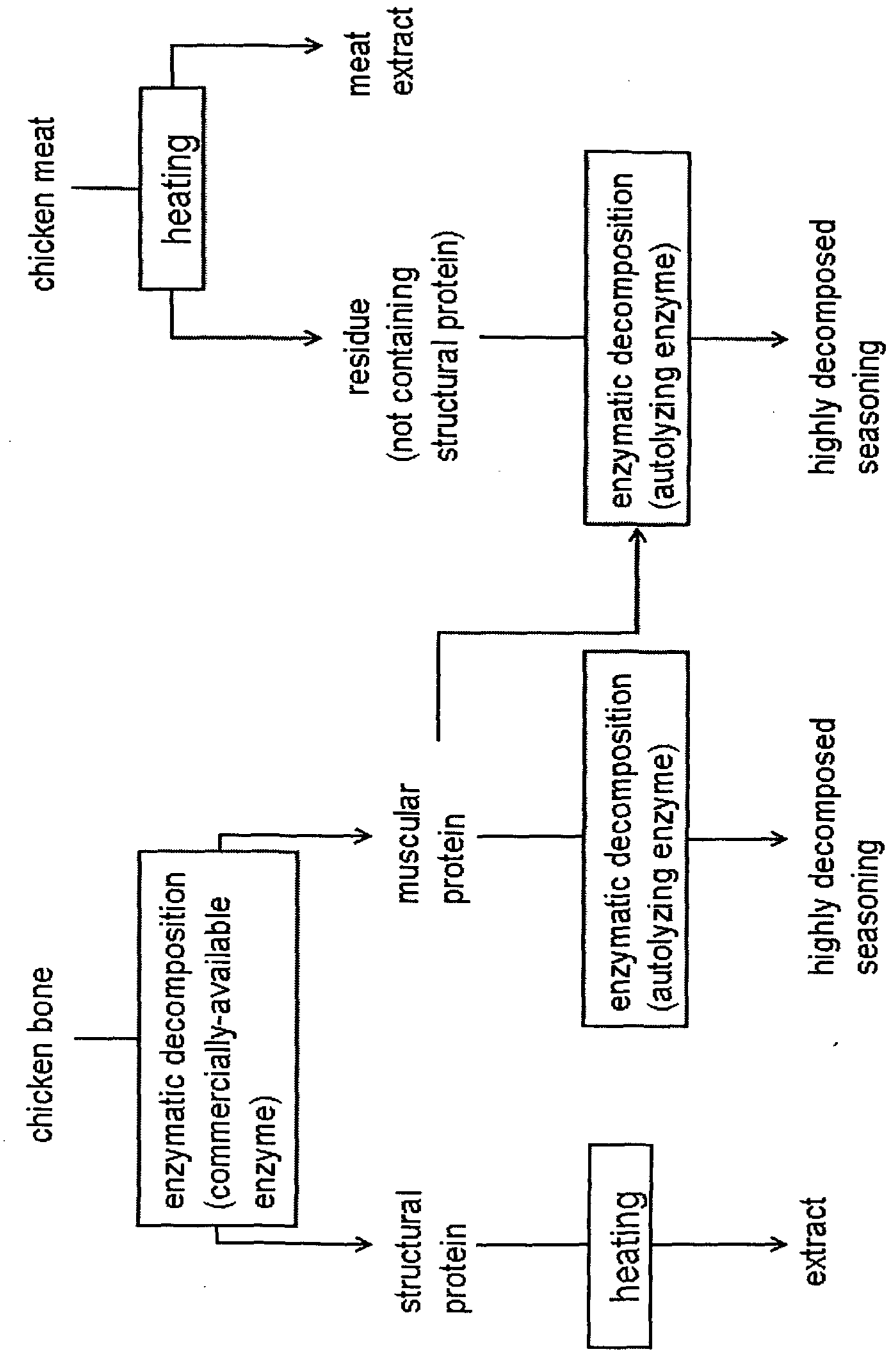
chicken meat
heating
meat extract
residue (not containing structural protein)
enzymatic decomposition (autolyzing enzyme)
highly decomposed seasoning
chicken bone
enzymatic decomposition (commercially-available enzyme)
muscular protein
enzymatic decomposition (autolyzing enzyme)
highly decomposed seasoning
structural protein
heating
extract

**INTERNATIONAL SEARCH REPORT**

International application No.
PCT/JP2004/013443

A. CLASSIFICATION OF SUBJECT MATTER
Int.Cl[7] A23L1/221, A23L1/227, A23J3/04, A23J3/34, A23L1/313

According to International Patent Classification (IPC) or to both national classification and IPC

B. FIELDS SEARCHED

Minimum documentation searched (classification system followed by classification symbols)
Int.Cl[7] A23L1/221, A23L1/227, A23J3/04, A23J3/34, A23L1/313

Documentation searched other than minimum documentation to the extent that such documents are included in the fields searched

| | | | |
|---|---|---|---|
| Jitsuyo Shinan Koho | 1940-1996 | Jitsuyo Shinan Toroku Koho | 1996-2004 |
| Kokai Jitsuyo Shinan Koho | 1971-1995 | Toroku Jitsuyo Shinan Koho | 1994-2004 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 62-257360 A (Kazuharu OSAJIMA),<br>09 November, 1987 (09.11.87),<br>& US 4853231 A | 1-7,20-22<br>8-19,23,24 |
| X<br>Y | JP 35-16983 B (Shigeo SAGAYAMA),<br>16 November, 1960 (16.11.60),<br>(Family: none) | 1-7,20-22<br>8-19,23,24 |
| X<br>Y | JP 59-224665 A (Fumio NISHIKAWA),<br>17 December, 1984 (17.12.84),<br>(Family: none) | 1-7,20-22<br>8-19,23,24 |
| X<br>Y | JP 5-507624 A (ADV. HYDROLYZING SYS.),<br>04 November, 1993 (04.11.93),<br>& WO 91/18520 A & EP 535135 A<br>& US 5053234 A | 1-7,20-22<br>8-19,23,24 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 November, 2004 (24.11.04) | 14 December, 2004 (14.12.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.
PCT/JP2004/013443

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 7-508414 A (NOVO-NORDISK AS),<br>21 September, 1995 (21.09.95),<br>& WO 94/1003 A & EP 648079 A<br>& US 5532007 A | 1-7,20-22<br>8-19,23,24 |
| X<br>Y | JP 55-58081 A (CPC INT. INC.),<br>30 April, 1980 (30.04.80),<br>& DE 2841043 A | 1-7,20-22<br>8-19,23,24 |
| X<br>Y | JP 59-220168 A (Taiyo Fishery Co., Ltd.),<br>11 December, 1984 (11.12.84),<br>(Family: none) | 1-7,20-22<br>8-19,23,24 |
| Y | JP 6-165654 A (Asahi Breweries, Ltd.),<br>14 June, 1994 (14.06.94),<br>(Family: none) | 8-19,23,24 |

Form PCT/ISA/210 (continuation of second sheet) (January 2004)